# EUROPÄISCHE PATENTSCHRIFT

(11) **EP 0 667 763 B1**
(45) Veröffentlichungstag und Bekanntmachung des Hinweises auf die Patenterteilung: **17.12.1997**
(21) Anmeldenummer: 93923509.9
(22) Anmeldetag: 20.10.1993
(51) Int. Cl.: A61J 15/00

(54) **SET ZUR SCHAFFUNG EINES KÜNSTLICHEN MAGENEINGANGS**
SET FOR THE CREATION OF AN ARTIFICIAL STOMACH ENTRANCE
SET DE CREATION D'UNE ENTREE ARTIFICIELLE DE L'ESTOMAC

(30) Priorität: 27.10.1992 DE 4236210
(43) Veröffentlichungstag der Anmeldung: 23.08.1995
(73) Patentinhaber: MARX, Karl-Heinz, D-22045 Hamburg (DE)
(72) Erfinder: MARX, Karl-Heinz, D-22045 Hamburg (DE)
(74) Vertreter: Siemons, Norbert, Dr.-Ing. Patentanwälte Hauck, Graalfs, Wehnert, Döring, Siemons
(86) Internationale Anmeldenummer: EP9302901
(87) Internationale Veröffentlichungsnummer: WO9409747

(56) Entgegenhaltungen:
- WO-A-89/06529
- US-A- 4 393 873
- US-A- 4 863 438
- US-A- 4 944 732

## Beschreibung

Die Erfindung bezieht sich auf ein Set zur Schaffung eines künstlichen Mageneingangs.

Implantate zur Verwendung bei der perkutanen Nahrungszufuhr werden bei der intragastralen Langzeiternährung eingesetzt. Die intragastrale Langzeiternährung von Patienten ist immer dann erforderlich, wenn die Nahrungsaufnahme über Mund und Speiseröhre nicht mehr funktioniert. Dazu kann es z.B. infolge von inoperablen Tumorobstruktionen des oberen Gastrointestinaltraktes, aber auch nach ausgedehnten kieferchirurgischen Eingriffen oder infolge z.B. neurogener Schluckstörungen kommen.

In derartigen Fällen kann ein Implantat in einen durch die Bauchdecke zum Magen führenden Stichkanal eingesetzt, und dann durch das Implantat flüssige Nahrung zugeführt werden.

Die aus der Literatur bekannten oder bereits im Markt eingeführten Implantate besitzen in übereinstimmender Weise die Grundform eines Schlauches, der an seinem distalen Ende eine mit mindestens einer Öffnung versehene Erweiterung trägt. Im implantierten Zustand sitzt diese Erweiterung im Mageninnenraum und sichert das Implantat gegen ein Herausziehen.

An die distale Erweiterung schließt sich ein zentraler rohrförmiger Bereich an, der durch den Stichkanal nach außen führt. Am proximalen Ende des rohrförmigen Bereiches ist eine Öffnung vorgesehen, die durch z.B. einen Stopfen verschließbar ist. Außerdem kann hier ein Außenflansch vorgesehen sein, der das Implantat auf der Bauchdecke fixiert. Zur Nahrungszufuhr wird das Implantat an seinem proximalen Ende geöffnet und dann über einen Schlauch mit einer Nahrungsmittelpumpe verbunden.

In der distalen Erweiterung kann zusätzlich ein Rückschlagventil vorgesehen sein, das ein Zurücklaufen der Nahrung, bzw. Eindringen von Magensaft in das Implantat verhindert.

Es sind nun unterschiedliche Techniken bekannt, mit denen das Implantat in den Magen eingesetzt werden kann. Handelt es sich um eine Erstimplantierung, so wird in der Regel eine Punktionskanüle in den Magen eingestochen und dann durch die Punktionskanüle ein Führungsfaden in den Magen eingesetzt. Der Führungsfaden wird mit einer durch ein Gastroskop in den Magen gelegten Fremdkörperzange ergriffen und mitsamt dem Gastroskop aus dem Mund herausgezogen. Das Implantat wird dann mit dem Führungsfaden verbunden, durch Mund und Speiseröhre in den Magen gezogen und dort so in den Stichkanal eingesetzt, daß das distal erweiterte Ende des Implantates an der Mageninnenwand anliegt.

Die beschriebene Technik ist relativ aufwendig (und mit Belastungen für den Patienten verbunden) und wird in der Regel nur bei Erstimplantierungen eingesetzt, bei denen der Operateur noch nicht auf einen bereits existierenden, ausgeheilten Stichkanal zurückgreifen kann. Soll dagegen ein verbrauchtes Implantat gegen ein neues ausgetauscht werden, dann genügt es in der Regel, das Implantat etwas zu strecken und dann durch den bereits länger bestehenden, gegen Verschiebungen der Gewebeschichten untereinander durch Verwachsungen bzw. Verklebungen gesicherten Stichkanal herauszuziehen. Das neue Implantat läßt sich auf entgegengesetzte Weise in den Magen von außen einsetzen. Zum Spannen kann z.B. ein in das Implantat einführbarer Stab verwendet werden. Ein derartiges spannbares Implantat ist z.B. aus der US-PS 5 007 900 bekannt geworden.

Aus der US 4 863 438 ist ein Implantat bekannt, bei dem die distale Erweiterung pilzförmig ist und durch Einsetzen eines dornähnlichen Werkzeuges in das angrenzende Röhrchen gestrafft werden kann, um durch eine chirurgische Öffnung, so wie eine existierende Gastrotomie, die durch Laparotomie oder durch eine perkutante endoskopische Technik hergestellt ist, eingeführt zu werden. Wenn sich das distale Ende im Magen befindet, wird der Dorn herausgezogen und aufgrund der Elastizität beult sich das pilzförmige Ende aus, um das Implantat im Magen zurückzuhalten. Dieses Implantat ist in der Regel auf den Einsatz in bereits existierende, ausgeheilte Stichkanäle beschränkt.

Aus der US-PS 3 961 632 ist ein Verfahren bekannt, daß eine Erstimplantierung von außen erlaubt. Bei diesem Verfahren wird ein Spezialimplantat über die Punktionskanüle gezogen und Kanüle und Implantat gemeinsam in den Magen eingestochen. Die Kanüle wird dann herausgezogen, wobei das Implantat in dem Stichkanal verbleibt und in seinem distalen Bereich eine Erweiterung ausbildet.

Dieses bekannte System hat jedoch den Nachteil, daß es speziell geformte Implantate voraussetzt. Unter Umständen kann es auch bei dem Herausziehen der Kanüle aus dem Implantat zu Problemen kommen, zumal das Implantat auf der Kanüle nur durch Haftreibung unsicher gehalten ist.

Aufgabe der Erfindung ist es daher, ein Set zu schaffen, mit dem sich auf schonende Weise ein künstlicher Mageneingang schaffen läßt.

Gelöst wird die Aufgabe mit einem Set, das die Merkmale der Ansprüche 1 oder 2 besitzt. Vorteilhafte Ausgestaltungen des Sets sind in den Unteransprüchen angegeben.

Die Erfindung bezieht sich auf ein Set, mit dem sich ein künstlicher Mageneingang schaffen läßt. Ein derartiges Set besteht aus einem in einen Stichkanal einsetzbaren Trokar mit Trokarhülse und einem Implantat. Wesentliche Voraussetzung ist, daß der Innendurchmesser der Trokarhülse und der Außendurchmesser des gespannten oder gequetschten Implantates entsprechend aufeinander abgestimmt sind. Erfindungsgemäß wird danach ein Implantat in Verbindung mit einer Spann- oder Quetschvorrichtung vorgesehen, die den Durchmesser des Implantates über seine gesamte Länge reversibel reduziert. In gespanntem oder gequetschtem Zustand ist das Implantat zusammen mit der Vorrichtung in einer üblichen Trokarhülse frei verschiebbar.

Es liegt auf der Hand, daß sich ein derartiges Implantat optimal implantieren läßt. Es genügt, den Magen in einem geeigneten Bereich mit einem üblicherweise in der Laparoskopie verwendeten Trokar mit Trokarhülse zu punktieren. Nach Entfernung des Trokars kann dann das erfindungsgemäße Implantat durch die Trokarhülse mit seinem distalen Ende in den Magen eingeführt werden. Sobald dies geschehen ist, wird die Trokarhülse herausgezogen und die Spann- bzw. Quetschvorrichtung von dem Implantat gelöst. Das Implantat verformt sich dann in seinen Betriebszustand und ist nach Ausheilung des Stichkanals einsatzbereit.

Nach Anspruch 1 ist als Spannvorrichtung ein Streckstab vorgesehen, der zwischen Widerlagern am proximalen und distalen Ende des Implantates angeordnet ist. Zur Verformung des Implantates in seinen Betriebszustand genügt es, den proximalen Endbereich, der das Widerlager trägt, abzutrennen, worauf sich der Stab dann problemlos entfernen läßt. Eine derartige Ausgestaltung ist relativ kostengünstig und bedienungsfreundlich.

Es ist weiterhin aber auch möglich, das Implantat, wie in Anspruch 2 beschrieben, in eine längliche Kunststoffhülle einzupressen. Die Ausgestaltung ermöglicht eine besonders effektive Verringerung des Betriebsdurchmessers. Außerdem ist aus Sterilitätsgründen sowieso eine Hülle für das Implantat vorzusehen, die im vorliegenden Fall lediglich verstärkt werden müßte. Auch diese Ausgestaltung läßt sich daher besonders kostengünstig verwirklichen.

In dem Zusammenhang ist es weiterhin vorteilhaft, wenn die Kunststoffhülle gemäß Anspruch 3 eine Schwächungszone an ihrem distalen Ende aufweist, durch die sich das Implantat aus der Hülle herausdrücken läßt. In vorteilhafter Weise wird dazu das Innere der Hülle mit einer Gleitsubstanz beschichtet. Das Implantat wird zusammen mit der Hülle, wie oben beschrieben, in den Stichkanal eingesetzt. Dann wird das Implantat an seinem proximalen Ende mit einer geeigneten Vorrichtung, z.B. einem Stab, gegenüber einer Verschiebung in proximaler Richtung gesichert und die Hülle nach außen weggezogen. Relativ gesehen wird das Implantat dabei, beginnend mit seinem distalen Ende aus der Hülle herausgedrückt und kann dann in dem Maße, in dem es von der Hülle freigegeben wird, seine Betriebsform einnehmen.

Alternativ ist es auch gemäß Anspruch 4 möglich, eine Hülle mit einem Aufreißfaden vorzusehen. Mit dem Aufreißfaden kann die Hülle in Längsrichtung geöffnet und dann nach oben abgezogen werden.

Eine weitere vorteilhafte Ausgestaltung der Erfindung betrifft Anspruch 5. Bei diesem Implantat bildet sich bei Verformung in den Betriebszustand ein proximaler Außenflansch aus, mit dem das Implantat auf der Bauchdecke fixiert wird. Das Implantat wird dann auf der Magenseite durch die distale Erweiterung und auf der Außenseite durch den Flansch sicher in dem Stichkanal fixiert. Zur Anpassung an abweichende Patientenerfordernisse werden Implantate mit unterschiedlich langen rohrförmigen Bereichen vorgesehen, von denen dann ein geeignetes ausgewählt wird. Vor der Implantierung muß dazu lediglich mit einer gängigen Technik die Länge des Stichkanals ausgemessen werden.

Zusätzlich kann gemäß einer Ausgestaltung nach Anspruch 6 aber auch eine unterhalb des Flansches an dem rohrförmigen Bereich anordenbare Halteplatte vorgesehen werden, die eine noch bessere Fixierung des Implantates auf der Bauchdecke gewährleistet. Andererseits kann man aber auch Halteplatten in unterschiedlichen Dicken vorrätig halten und so gegebenenfalls durch Auswahl einer geeigneten Halteplatte ein nicht ganz in der Länge passendes Implantat an die Länge des Stichkanals anpassen.

Nach einer weiteren Ausgestaltung gemäß Anspruch 7 kann an einer derartigen Halteplatte ein Stopfen angelenkt sein, mit dem sich die proximale Öffnung des rohrförmigen Bereiches verschließen läßt. Vorteilhaft an dieser Ausgestaltung ist vor allem, daß der Stopfen nachträglich montiert wird. Der in der Regel aus einem härteren Material bestehende Stopfen ist daher in dem durchmesserreduzierten Implantat noch nicht enthalten, was gegebenenfalls die Quetschung oder die Spannung erleichtert.

Eine weitere Ausgestaltung der Erfindung betrifft die Ausbildung eines Rückschlagventils in der distalen Erweiterung. Übliche Rückschlagventile arbeiten mit einer Klappe, die im Bereich der Einmündung des zentralen rohrförmigen Bereiches in der Erweiterung angeordnet ist. Diese Klappe öffnet bei distaler und schließt bei proximal gerichteter Strömung. Nachteilig an einer derartigen Klappe ist, daß sie bei der Spannung oder Quetschung des Implantats die Durchmesserreduzierung behindert und einen die Funktion beeinträchtigenden Schaden nehmen kann. Daher sieht eine Ausgestaltung gemäß Anspruch 8 vor, daß das Rückschlagventil eine nach innen weisende Einstülpung ist, die sich federnd gegen die Einmündung des zentralen rohrförmigen Teiles in die Erweiterung anlegt. Ein derartiges Rückschlagventil läßt sich ohne weiteres mit gängigem Kunststoffmaterial verwirklichen und ist besonders kostengünstig und bedienungssicher, da es die Durchmesserreduzierung nicht behindert und nicht beschädigt werden kann.

In einem derartigen Set können Meßeinrichtungen zur Bestimmung der Länge des Stichkanales vorgesehen sein, die es dem Operateur ermöglichen, das Implantat in geeigneter Länge auszuwählen. Vorteilhaft sind dazu die Merkmale des Anspruches 9 vorgesehen. Diese Meßeinrichtung erlaubt es, vor Einsetzen des Implantates durch die bereits verlegte Trokarhülse einen Taster einzuführen, dessen distalen Anschlag vor dem Ende der Trokarhülse seitlich auszufahren und sodann Anschlag und Trokarhülse soweit nach außen zu ziehen, daß der Anschlag an der Magenwand anliegt. Die Trokarhülse liegt dann mit ihrem distalen Ende bündig mit der Magenwand, so daß auf der Skala der Trokarhülse die Länge des Stichkanales genau abgelesen werden kann. Als Taster sind mechanische Konstruktionen einsetzbar, bei denen der Anschlag beispielsweise als Klapphebel ausgebildet ist oder auch Konstruktionen, die etwa nach Art eines Ballonkatheters ausgebildet sind.

Die Erfindung soll im folgenden an Hand mehrerer, unterschiedliche Ausführungsbeispiele zeigender Abbildungen näher erläutert werden.

Dabei zeigen die
- Fig. 1 bis 3: ein erstes Ausführungsbeispiel des erfindungsgemäßen Implantates in unterschiedlichen Stadien der Implantierung,
- Fig. 4 und 5: ein zweites Ausführungsbeispiel während der Implantierung,
- Fig. 6: ein drittes Ausführungsbeispiel der Erfindung im implantierten Zustand und
- Fig. 7 und 8: zwei Ausführungsformen einer Meßeinrichtung zur Bestimmung der Länge des Stichkanales.

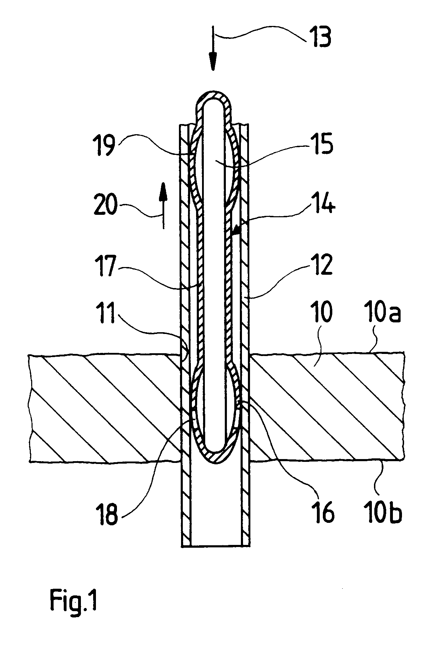

In Figur 1 ist ein Schnitt durch die Bauchdecke und grob schematisch Patientengewebe 10 dargestellt, das nach oben hin durch die Bauchhaut 10a und nach unten hin durch die Magenwand 10b begrenzt wird. Mittels nicht gezeigter Vorrichtungen, z.B. einem üblichen Trokar, ist ein Stichkanal 11 durch das Gewebe 10 in den Magen des Patienten geschaffen worden. In diesem Stichkanal 11 ist eine übliche Trokarhülse 12 eingesetzt, durch die, wie in Fig. 1 durch den Pfeil 13 dargestellt, ein Implantat 14 in Richtung des Magens eingeschoben wird. In dem Implantat 14 ist ein Streckstab 15 unter Spannung angeordnet, der das Implantat 14 in eine axial gelängte Form bringt und dergestalt seinen Durchmesser verringert.

Das Implantat 14 besitzt eine distale Erweiterung 16, die sich an einem zentralen rohrförmigen Bereich 17 anschließt. In der distalen Erweiterung 16 sind Öffnungen 18 vorgesehen, durch die flüssige Nahrung in den Magen eintreten kann. Schließlich besitzt das Implantat 14 in seinem proximalen Endbereich eine an den zentralen rohrförmigen Bereich 17 anschließende flanschartige Erweiterung 19.

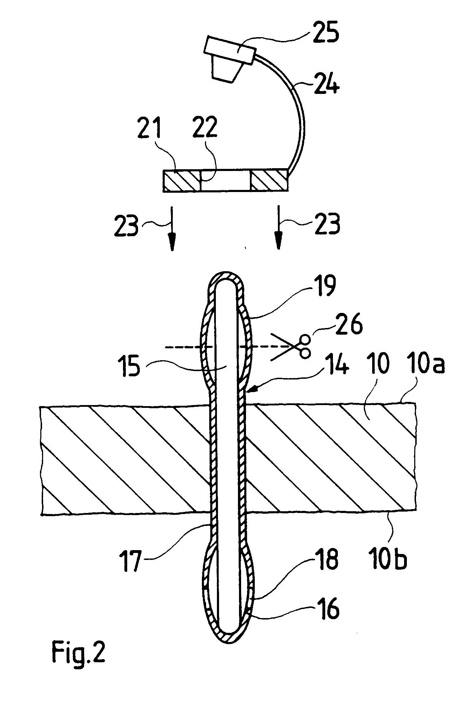

Um den Übergang zu Fig. 2 besser zu verstehen, ist in Figur 1 ein Pfeil 20 dargestellt, mit dem angedeutet werden soll, daß die Trokarhülse 12 aus dem Stichkanal 11 herausgenommen wird.

Fig. 2 zeigt den Zustand, in dem die Trokarhülse 12 entfernt worden ist. Das Gewebe 10 schließt sich nun dicht um das Implantat 14, das im übrigen noch unverändert gegenüber der in Fig. 1 gezeigten Form ist.

Zu diesem Zeitpunkt wird eine Halteplatte 21, die eine zentrale Öffnung 22 aufweist, in Richtung der Pfeile 23 über das proximale Ende des Implantates 14 auf die Bauchhaut 10a des Patienten aufgesetzt. An der Halteplatte 21 ist über einen biegsamen Steg 24 ein Stopfen 25 angelenkt.

Nachdem die Halteplatte 21 in Position gebracht worden ist, wird bei der mit Scherensymbol 26 bezeichneten Linie das proximale Ende des Implantates 14 abgeschnitten und der Streckstab 15 entfernt.

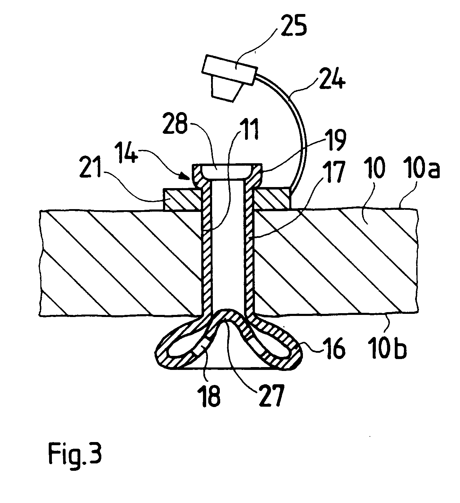

Nach Entfernung des Streckstabes 15 verformt sich das Implantat 14 in seinen Betriebszustand, wie in Fig. 3 dargestellt. Der zentrale rohrförmige Bereich 17 erstreckt sich nun über die Länge des Stichkanals 11. Gegen ein Herausziehen ist das Implantat 14 durch die distale Erweiterung 16 gesichert, die sich von innen gegen die Magenwand 10b anlegt. Man erkennt nun, daß die distale Erweiterung 16 eine Einstülpung 27 besitzt, die elastisch gegen die Öffnung des zentralen rohrförmigen Bereiches 17 liegt. Die Einstülpung 27 dient als Rückschlagventil und verhindert, daß Magensaft oder flüssige Nahrung nach außen austritt.

Man erkennt weiterhin, daß die über den proximalen Endbereich des Implantates 14 geschobene Halteplatte 21 durch die flanschartige Erweiterung 19 auf der Bauchdecke verriegelt wird. Der sichere Sitz des Implantates 14 ist also gewährleistet. Weiterhin wurde im Zuge der Entfernung des Streckstabes 15 aus dem Implantat 14 gleichzeitig eine proximale Öffnung 28 geschaffen, die mittels des Stopfens 25 verschließbar ist.

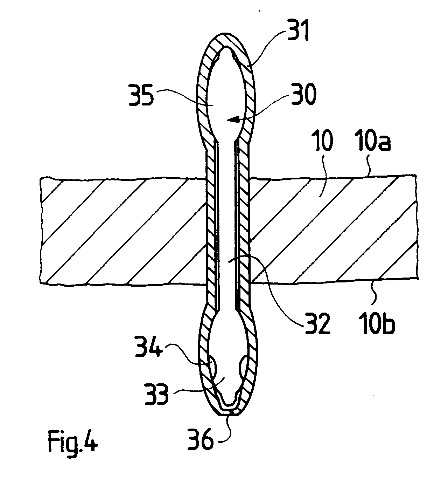

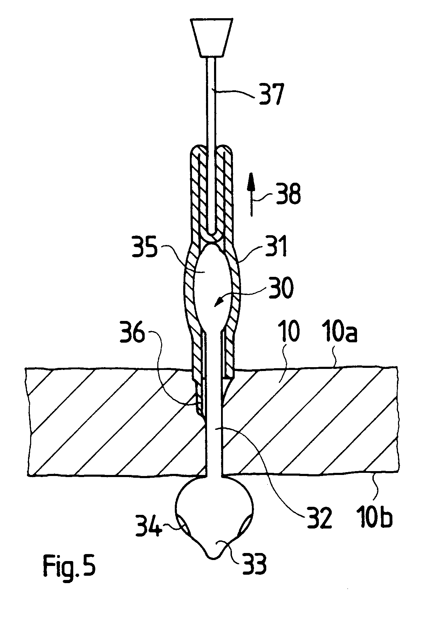

In den Figuren 4 und 5 ist ein weiteres Ausführungsbeispiel der Erfindung dargestellt. In Fig. 4 erkennt man ein in eine Kunststoffhülle 31 eingepreßtes Implantat 30, das in das Gewebe 10 eingesetzt ist. Der gezeigte Zustand entspricht im wesentlichen dem in Fig. 2, d.h. die zur Implantierung verwendete Trokarhülse wurde bereits entfernt.

Das Implantat 30 besitzt einen zentralen rohrförmigen Bereich 32, eine mit Öffnungen 34 versehene distale Erweiterung 33 und eine proximale Erweiterung 35. In diesem Fall dient zur Durchmesserreduzierung des Implantates 30 die Kunststoffhülle 31, die an ihrem distalen Ende mit einer Schwächungszone 36 versehen ist.

In Fig. 5 ist nun dargestellt, wie das Implantat 30 durch diese Schwächungszone 36 aus der Kunststoffhülle 31 entfernt wird und seinen Betriebszustand einnimmt. Im dargestellten Fall wird eine stabförmige Vorrichtung 37 auf das proximale Ende des Implantates 30 aufgesetzt und die Kunststoffhülle 31 in Richtung des Pfeiles 38 nach außen von dem Implantat abgezogen. In dem Maße, in dem die Hülle 31 das Implantat 30 freigibt, nimmt dieses seine Betriebsform ein.

Auch hier wird das Implantat dann im Bereich der proximalen Erweiterung 35 durch einen Schnitt geöffnet und gegebenenfalls mit Hilfe einer Halteplatte, wie in Fig. 3 gezeigt, verriegelt.

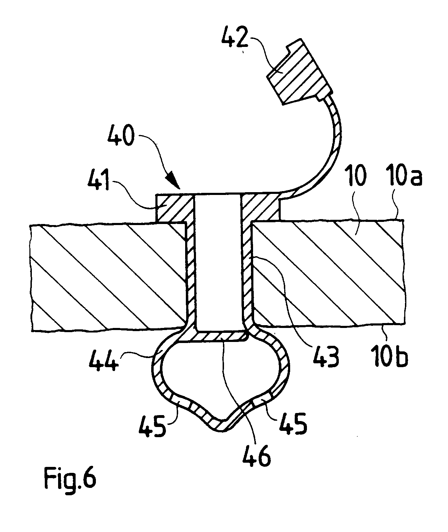

Ein etwas anderes Ausführungsbeispiel zeigt Fig. 6. Man erkennt ein Implantat 40, an dem eine proximale Erweiterung 41 bereits in Form einer Halteplatte ausgebildet ist. Weiterhin ist bereits an dieser Halteplatte 41 ein Stopfen 42 angelenkt. Eine derartige einstückige Version erspart gegebenenfalls bei der Operation einige Handgriffe, hat jedoch den Nachteil, daß die Durchmesserreduzierung schwieriger ist.

An die plattenartige Erweiterung 41 schließt sich in gewohnter Weise ein zentraler rohrförmiger Bereich 43 an, der in eine distale Erweiterung 44 einmündet. Die distale Erweiterung 44 besitzt Öffnungen 45. Ein als Klappe ausgebildetes Rückschlagventil 46 zwischen zentralem rohrförmigen Bereich 43 und distaler Erweiterung 44 sorgt schließlich dafür, daß der Durchfluß nur in der gewünschten Richtung von außen nach innen stattfinden kann.

Die gezeigten Ausführungsbeispiele geben die Erfindung nur beispielhaft wieder. So ist es z.B. auch möglich, ein Implantat gleichzeitig mit einem Streckstab zu spannen und mit einer Kunststoffhülle zu umgeben. Dies kann z.B. aus Gründen der Sterilität von Vorteil sein. Darüber hinaus kann man die Öffnung der Kunststoffhülle auch auf andere Weise, z.B. mittels eines Aufreißfadens vorsehen. Das Implantat kann schließlich einzeln oder auch im Set mit einer vom Durchmesser her geeigneten Trokarhülse angeboten werden. Zu diesem Set können weiterhin auch Vorrichtungen zur Bestimmung der Länge des Stichkanales und zur Schaffung des Zuganges gehören. Diese Instrumente gehören der üblichen Medizintechnik an und bedürfen daher keiner weiteren Erläuterung.

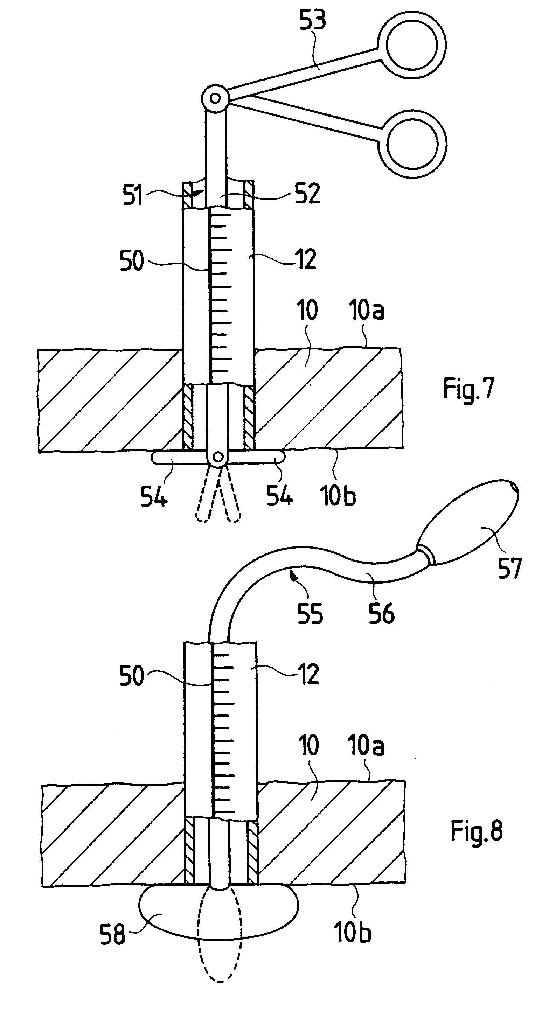

Vorteilhafte Ausgestaltungen von Meßeinrichtungen zur Bestimmung der Länge des Stichkanales zeigen die Figuren 7 und 8 in zwei Ausführungsformen.

Fig. 7 zeigt in einem Schnitt, entsprechend Figur 1, die Bauchdecke 10, 10a, 10b mit der Trokarhülse 12, die in diesem Falle als Besonderheit, wie in dem nicht geschnittenen Bereich der Trokarhülse zu sehen ist, eine Längenskala 50 aufweist.

Durch die Trokarhülse 12 ist ein Taster 51 eingesteckt, der nach Art einer üblichen Laparoskopschere ausgebildet ist mit langgestrecktem Schaft 52 und von einem Scherengriff 53 betätigbaren, an seinem distalen Ende angelenkten Klappbranchen 54. Diese können in der gestrichelt dargestellten zusammengeklappten Stellung durch das Innere der Trokarhülse 12 bis in den Magen eingeführt werden und sodann durch Betätigung des Scherengriffes 53 in die ausgezogen dargestellte Stellung nach außen geklappt werden. Wird in dieser Stellsung nun der Taster 51 nach außen gezogen, so legen sich die Klappbranchen 54 gegen das distale Ende der Trokarhülse 12 und ziehen diese mit nach außen, bis die Klappbranchen 54 gegen die Magenwand 10b liegen. Die Trokarhülse 12 liegt nun mit ihrem distalen Ende bündig mit der Magenwand 10b. Auf der Längenskala 50 kann die Dicke der Bauchdecke, also des Gewebes 10, zwischen Bauchhaut 10a und Magenwand 10b abgelesen werden.

Es kann nun, entsprechend der gemessenen Länge des Stichkanales, ein Implantat in geeigneter Länge ausgesucht werden, damit es verschiebungssicher im Stichkanal anordenbar ist.

Figur 8 zeigt die Bauchdecke 10, 10a, 10b und die Trokarhülse 12 der Figur 7 und einen Taster 55 anderer Ausführungsform.

Der Taster 55 besteht aus einem langgestreckten elastischen Schlauch 56, an dessen proximalem Ende eine Pumpeinrichtung für ein flüssiges Medium angeordnet ist. Im dargestellten Falle handelt es sich um einen Handpumpball 57.

Am distalen Ende des Schlauches 56 sitzt ein aufblasbarer Ballon 58, der aus der gestrichelt dargestellten entspannten Stellung, in der er durch die Trokarhülse 12 einführbar ist, in die ausgezogen dargestellte aufgeblasene Stellung aufpumpbar ist. In der aufgeblasenen Stellung überragt er seitlich die Trokarhülse 12 und kann durch Ziehen am Schlauch 56 das distale Ende der Trokarhülse 12 in bündige Lage mit der Magenwand 10b bringen, ebenso wie dies der Taster 51 der Figur 7 tut.

Die beiden Taster 51, 55 der Figuren 7 und 8 werden zur Längenbestimmung des Stichkanales in der beschriebenen Weise eingesetzt und dann wieder aus der Trokarhülse entfernt, bevor das Implantat eingesetzt wird. Zum Entfernen der Taster 51 bzw. 55 werden die an ihrem distalen Ende vorgesehenen Anschläge 54 bzw. 58 wieder durch Zusammenklappen bzw. Druckentlasten in eine derart durchmesserreduzierte Stellung gebracht, daß sie durch die Trokarhülse nach außen ziehbar sind.

## Patentansprüche

1. Set zur Schaffung eines künstlichen Mageneinganges, bestehend aus einem in den Magen durch einen Stichkanal (11) von außen einsetzbaren Trokar mit Trokarhülse (12) und einem Implantat (14, 30, 40) aus elastisch verformbarem Material, mit einer in den Magen einsetzbaren, distalen Erweiterung (16, 33, 44), die mindestens eine Öffnung aufweist und einem proximal daran anschließenden zentralen rohrförmigen Bereich (17, 32, 43), wobei das Implantat (14, 30, 40) eine lösbare Spannvorrichtung (15) aufweist, die den Durchmesser des Implantates (14, 30, 40) über seine gesamte Länge überall dort reversibel reduziert, wo anderenfalls eine freie Verschiebung durch die Trokarhülse (12) verhindert würde, wodurch es zusammen mit der Vorrichtung in der Trokarhülse (12) frei verschiebbar ist, wobei als Spannvorrichtung ein Streckstab (15) zwischen Widerlagern am proximalen und distalen Ende des Implantats (14) angeordnet ist.

2. Set zur Schaffung eines künstlichen Mageneinganges, bestehend aus einem in den Magen durch einen Stichkanal (11) von außen einsetzbaren Trokar mit Trokarhülse (12) und einem Implantat (14, 30, 40) aus elastisch verformbarem Material, mit einer in den Magen einsetzbaren, distalen Erweiterung (16, 33, 44), die mindestens eine Öffnung aufweist und einem proximal daran anschließenden zentralen rohrförmigen Bereich (17, 32, 43), wobei das Implantat (14, 30, 40) eine lösbare Quetschvorrichtung (31) aufweist, die den Durchmesser des Implantates (14, 30, 40) über seine gesamte Länge überall dort reversibel reduziert, wo anderenfalls eine freie Verschiebung durch die Trokarhülse (12) verhindert würde, wodurch es zusammen mit der Vorrichtung in der Trokarhülse (12) frei verschiebbar ist, wobei als Quetschvorrichtung eine rundum geschlossene längliche elastische Hülle (31) vorgesehen ist, in die das Implantat (30) eingepreßt ist.

3. Set nach Anspruch 2, bei dem die Hülle (31) im Inneren mit einer Gleitsubstanz beschichtet ist und an ihrem distalen Ende eine Schwächungszone (36) besitzt, durch die sich unter leichtem Druck das Implantat (30) in distaler Richtung aus der Hülle (31) herausdrücken läßt.

4. Set nach Anspruch 2, bei dem die Hülle einen im wesentlichen über ihre Gesamtlänge erstreckten Aufreißbereich mit einem darin eingebetteten Aufreißfaden aufweist.

5. Set nach einem der Ansprüche 1 bis 4, bei dem an dem proximalen Ende des rohrförmigen Bereichs (17, 32, 43) ein Außenflansch (19, 35, 41) vorgesehen ist, der sich nach Entfernen der Spann- oder Quetschvorrichtung ausbildet und als Fixierung des Implantats (14, 30, 40) auf der Bauchoberfläche dient.

6. Set nach einem der Ansprüche 1 bis 5, bei dem eine an dem rohrförmigen Bereich (17) zwischen Außenflansch (19) und Bauchdecke anordenbare Halteplatte (21) vorgesehen ist.

7. Set nach Anspruch 6, bei dem an der Halteplatte (21) über einen biegsamen Steg (24) ein Stopfen (25) angelenkt ist, der als Verschluß für eine proximale Öffnung (28) des Implantats (14) dient.

8. Set nach einem der Ansprüche 1 bis 7, bei dem in seiner distalen Erweiterung ein Rückschlagventil ausgebildet ist und die distale Erweiterung (16) als Ventil eine nach innen weisende Einstülpung (27) besitzt, die federnd gegen die Einmündung des zentralen rohrförmigen Teiles (17) in die Erweiterung (16) anliegt.

9. Set nach einem der Ansprüche 1 bis 8, bei dem eine Meßeinrichtung zur Bestimmung der Länge des Stichkanales (11) vorgesehen ist, die eine Längenskala (50) auf der Außenseite der Trokarhülse sowie einen Taster (51, 55) aufweist, der durch die Trokarhülse (12) einsetzbar ist und an seinem distalen Ende einen Anschlag (54, 58) aufweist, der vor dem distalen Ende der Trokarhülse (12) seitlich ausfahrbar ist.

## Claims

1. A set for the creation of an artificial stomach entrance, consisting of a trocar with a trocar sleeve (12) able to be inserted into the stomach through a piercing channel (11) from the outside, and an implant (14, 30, 40) of elastically deformable material, with a distal extension (16, 33, 44) which can be inserted into the stomach and which comprises at least one opening, and proximally connecting to this, a central tubular region (17, 32, 44), wherein the implant (14, 30, 40) comprises a releasable fastening device (15) which reversibly reduces the diameter of the implant (14, 30, 40) over its whole length everywhere where otherwise a free displacement is prevented by the trocar sleeve, by which means the implant together with the device is freely displaceable in the trocar sleeve (12), wherein as a fastening device an extending rod (15) is arranged between counter bearings at the proximal and distal end of the implant (14).

2. A set for the creation of an artificial stomach entrance, consisting of a trocar with a trocar sleeve (12) able to be inserted into the stomach through a piercing channel (11) from the outside, and an implant (14, 30, 40) of elastically deformable material, with a distal extension (16, 33, 44) which can be inserted into the stomach and which comprises at least one opening, and proximally connecting to this, a central tubular region (17, 32, 44), wherein the implant (14, 30, 40) comprises a releasable squeezing device (15) which reversibly reduces the diameter of the implant (14, 30, 40) over its whole length everywhere where otherwise a free displacement is prevented by the trocar sleeve, by which means the implant together with the device is freely displaceable in the trocar sleeve (12), wherein as a squeezing device an all round closed elongate elastic casing (31) is provided into which the implant (30) can be pressed.

3. A set according to claim 2, in which the casing (31) on the inside is coated with a gliding substance and at its distal end has a weakening zone (36) by which means with a slight pressure the implant (39) may be pressed out of the casing (31) in the distal direction.

4. A set according to claim 2, in which the casing comprises a tear region extending essentially over its whole length with a tear thread embedded therein.

5. A set according to one of claims 1 to 4, in which at the proximal end of the tubular region (17, 32, 43) there is provided an outer flange (19, 35, 41) which develops after removing the fastening or squeezing device and serves the fixation of the implant (14, 30, 40) on the surface of the abdomen.

6. A set according to one of claims 1 to 5, in which there is provided a holding plate (21) arrangeable on the tubular region (17) between the outer flange (19) and the abdominal cover.

7. A set according to claim 6 in which a plug (25) is coupled to the holding plate (21) via a bendable web (24), the plug serving as a closure for the proximal opening (28) of the implant (14).

8. A set according to one of claims 1 to 7, in which in its distal extension there is formed a return valve and the distal extension (16) as a valve has an inversion (27) facing inwardly which resiliently rests against the mouth of the central tubular part (17) in the extension (16).

9. A set according to one of claims 1 to 8, in which there is provided a measuring device for determining the length of the piercing channel (11), and comprises a length scale (50) on the outer side of the trocar sleeve as well as a probe (51, 55) which can be inserted through the trocar sleeve (12) and which, at its distal end comprises a stop (54, 58) laterally extendable in front of the distal end of the trocar sleeve (12).

## Revendications

1. Système destiné à créer une entrée d'estomac artificielle, comprenant d'une part un trocart avec canule de trocart (12) qui peut être inséré depuis l'extérieur dans l'estomac par un canal percutané (11) et d'autre part un implant (14, 30, 40) en matériau élastique déformable présentant un renflement (16, 33, 44) distal qui peut être mis en place dans l'estomac et qui comporte au moins une ouverture et une zone centrale de forme tubulaire (17, 32, 43) s'y raccordant du côté proximal, l'implant (14, 30, 40) présentant un dispositif de tension amovible (15), qui réduit de manière réversible le diamètre de l'implant (14, 30, 40) sensiblement sur la totalité de sa longueur partout où à défaut une translation libre à travers la canule de trocart (12) serait empêchée, ce qui le rend capable de translation libre dans la canule de trocart (12) avec le dispositif, une tige d'extension (15) étant montée à titre de dispositif de tension, entre des butées aux extrémités proximale et distale de l'implant (14).

2. Système destiné à créer une entrée d'estomac artificielle, comprenant d'une part un trocart avec canule de trocart (12) qui peut être inséré depuis l'extérieur dans l'estomac par un canal percutané (11) et d'autre part un implant (14, 30, 40) en matériau élastique déformable présentant un renflement (16, 33, 44) distal qui peut être mis en place dans l'estomac et qui comporte au moins une ouverture et une zone centrale de forme tubulaire (17, 32, 43) pouvant s'y raccorder côté proximal, l'implant (14, 30, 40) présentant un dispositif de compression amovible (31), qui réduit de manière réversible le diamètre de l'implant (14, 30, 40) sensiblement sur la totalité de sa longueur partout où à défaut une translation libre à travers la canule de trocart (12) serait empêchée, ce qui le rend capable de translation libre dans la canule de trocart (12) avec le dispositif, une enveloppe (31) élastique allongée complètement fermée, dans laquelle l'implant est comprimé, étant prévue à titre de dispositif de compression.

3. Système selon la revendication 2, dans lequel l'enveloppe (31) est enduite intérieurement d'une substance lubrifiante et présente à son extrémité distale une zone de faiblesse (36) par laquelle, sous l'effet d'une légère pression, il est possible d'expulser l'implant (30) hors de l'enveloppe (31), dans le sens distal.

4. Système selon la revendication 2, dans lequel l'enveloppe présente une zone à déchirer s'étendant sensiblement sur la totalité de sa longueur, dans laquelle est incorporé un fil à déchirer.

5. Système selon l'une des revendications 1 à 4, dans lequel une bride extérieure (19, 35, 41), qui se forme une fois que l'on retire le dispositif de tension ou de compression et qui sert à fixer l'implant (14, 30, 40) sur la surface de l'abdomen, est prévue à l'extrémité proximale de la zone de forme tubulaire (17, 32, 43).

6. Système selon l'une des revendications 1 à 5, dans lequel est prévue une plaque de maintien (21) qui est montée sur la zone de forme tubulaire (17) entre la bride extérieure (19) et la paroi abdominale.

7. Système selon la revendication 6, dans lequel un bouchon (25), qui sert à obturer une ouverture proximale (28) de l'implant (14), est articulé sur la plaque de maintien (21) par l'intermédiaire d'une tige flexible (24).

8. Système selon l'une des revendications 1 à 7, dans le renflement distal duquel est formé un clapet anti-retour et dont le renflement distal (16) comporte à titre de clapet une concavité (27) orientée vers l'intérieur qui prend appui de manière élastique contre l'embouchure de la partie centrale de forme tubulaire (17) dans le renflement (16).

9. Système selon l'une des revendications 1 à 8, dans lequel un dispositif de mesure est prévu pour déterminer la longueur du canal percutané (11), qui comporte un échelle de mesure de longueur (50) sur le côté extérieur de la canule de trocart ainsi qu'un palpeur (51, 55) pouvant être inséré à travers la canule de trocart (12) et présentant à son extrémité distale un butoir (54, 58) qui peut être déployé en amont de l'extrémité distale de la canule de trocart (12).
